# EUROPEAN PATENT SPECIFICATION

(11) **EP 3 508 849 B1**
(45) Date of publication and mention of the grant of the patent: **19.04.2023**
(21) Application number: 17846588.6
(22) Date of filing: 30.08.2017
(51) Int. Cl.: G01N 33/543, G01N 33/569

(54) **ANTIBODY MEASUREMENT METHOD USING ANTIGEN-CARRYING INSOLUBLE CARRIER PARTICLES ON WHICH ANTIGEN IS IMMOBILIZED BY DIFFERENT METHODS, AND REAGENT FOR ANTIBODY MEASUREMENT**
ANTIKÖRPERMESSVERFAHREN MIT VERWENDUNG VON ANTIGENTRAGENDEN NICHTLÖSLICHEN TRÄGERTEILCHEN, AUF DENEN DAS ANTIGEN DURCH UNTERSCHIEDLICHE VERFAHREN IMMOBILISIERT IST, UND REAGENZ ZUR ANTIKÖRPERMESSUNG
PROCÉDÉ DE MESURE D'ANTICORPS METTANT EN OEUVRE DES PARTICULES DE SUPPORT INSOLUBLES PORTEUSES D'ANTIGÈNE TEL QU'UN ANTIGÈNE EST FIXÉ PAR UN PROCÉDÉ DIFFÉRENT, ET RÉACTIF POUR MESURE D'ANTICORPS

(30) Priority: 31.08.2016 JP 2016168772
(43) Date of publication of application: 10.07.2019
(73) Proprietor: Eiken Kagaku Kabushiki Kaisha, Tokyo 110-8480 (JP)
(72) Inventor: UENO Yusuke, Shimotsuga-gun Tochigi 329-0114 (JP); KIKUCHI Tatsunori, Shimotsuga-gun Tochigi 329-0114 (JP); MURAKAMI Yusuke, Shimotsuga-gun Tochigi 329-0114 (JP)
(74) Representative: Henkel & Partner mbB
(86) International application number: PCT/JP2017/031218
(87) International publication number: WO 2018/043584

(56) References cited:
- EP-A1- 2 287 609
- EP-A2- 0 295 402
- WO-A1-2014/073464
- JP-A- H10 239 317
- JP-A- 2001 507 447
- JP-A- 2013 536 952
- PEURA, JM et al.: "COVALENT BINDING OF PROTEINS TO ACETAL-FUNCTIONALIZED LATEXES. II. COLLOIDAL STABILITY AND IMMUNOREACTIVITY", JOURNAL OF COLLOID AND INTERFACE SCIENCE, vol. 201, 1998, pages 139-145, XP055472142, DOI: doi:10.1006/jcis.1998.5389

## Description

### Technical Field

The present invention relates to a method for assaying an antibody by using, in combination, antigen-immobilizing insoluble carrier particles such as, for example, antigen-immobilizing latex particles, on which the antigen is immobilized through different processes. The present invention further relates to an antibody assay reagent and an assay kit.

### Background Art

Currently, among diagnostic reagents containing insoluble carrier particles, a diagnostic agent using a latex agglutination assay as a type of immunoassays, is particularly used widely.

The latex agglutination assay involves use of latex particles having an antigen or an antibody immobilized thereon in a liquid phase for formation of an assay system for detection of a corresponding antibody or antigen. This assay is based on the mechanism by which latex particles agglutinate through formation of immune complexes. In this assay, the degree of agglutination can be determined by visual observation, or an increase in turbidity can be optically determined on the basis of a change in absorbance or scattered light intensity (hereinafter this assay may be referred to as "latex assay": Patent Document 1).

Meanwhile, a sandwich immunoassay (e.g. ELISA) achieves accurate measurement and can be used as a standard method, but such an immunoassay involves, during measurement, a washing process for removing a component (other than a substance to be assayed) contained in an assay sample or a component contained in an assay reagent. Therefore, such an immunoassay has problems in that it requires a long time and complicated operation for assay, and thus it is not suitable for assay of many test samples within a short period of time.

In contrast, the latex assay involves a simple operation and is readily applicable to a general-purpose biochemical automatic analyzer that is used widely in clinical examinations. Thus, the latex assay can achieve assay of many test samples within a short period of time.

Accordingly, the latex assay is used for many clinical examinations.

Patent Document 2 discloses an antigen peptide that can be used as an antibody test material for anti-malaria protozoa antibodies, a test agent and a test kit comprising the antibody test material, and a method of testing for an infection with malaria protozoa in a subject using the antibody test material. The antibody test material comprises, as an active ingredient, an antigen peptide having a total of 19 amino acids and comprising an common amino acid sequence part shared between *falciparum* malaria-derived LDH and *vivax* malaria-derived LDH. The antigen peptide can be bound to, immobilized on or adsorbed to the surface of a solid-phase material such as polymeric microparticles.

Patent Document 3 discloses an immunodiagnostic test kit and a method for accurately and efficiently detecting *Helicobacter pylori* infection in a subject. The immunodiagnostic test kit comprises, among others, one or more *Helicobacter pylori* type-common antigens such as *Helicobacter pylori* urease, and one or more purified type-specific *Helicobacter pylori* Type I antigens such as an *Helicobacter pylori* Type I VacA polypeptide and an *Helicobacter pylori* Type I CagA polypeptide. Preferably, the one or more *Helicobacter pylori* type-common antigens are provided in an *Helicobacter pylori* lysate.

Patent Document 4 discloses insoluble carrier particles, a measurement reagent, a specimen analyzing tool, and an immunoturbidimetric assay capable of suppressing nonspecific aggregation even when the amount of antibody or antigen to be carried is increased. The insoluble carrier particles are produced by a method comprising at least one of the following sensitization reaction processes (A) and (B):
(A) bringing the antibody or the antigen into contact with the insoluble carrier particle in the presence of an amino acid with a charged polar side chain in a sensitization reaction solution, wherein a concentration of the amino acid with a charged polar side chain in the sensitization reaction solution is more than 0.1 mol/L and is equal to or less than 1 mol/L; and
(B) bringing the antibody or the antigen into contact with the insoluble carrier particle in the presence of an amino acid with a charged polar side chain in a sensitization reaction solution, wherein a latex particle for chemical bonding is used as the insoluble carrier particle.

### Prior Art Document

### Patent Document

Patent Document 1: JP S53-62826 A
Patent Document 2: WO 2014/073464 A1
Patent Document 3: JP 2001-507447 A
Patent Document 4: EP 2 287 609 A1

### Disclosure of the Invention

### Problems to be Solved by the Invention

An object of the present invention is to widen an antibody assay range and to enhance sensitivity and specificity of antibody assay employing insoluble carrier particles such as latex particles, on which an antigen is immobilized. In particular, an object of the present invention is to provide means for achieving good assay results even by use of insoluble carrier particles on which a mixture of a plurality of substances (e.g. a lysate derived from a microorganism) is immobilized.

In development of a diagnostic reagent containing insoluble carrier particles, the performance of the diagnostic reagent is greatly determined by the molecular weight of a substance to be immobilized on the insoluble carrier particles and the method for immobilizing the substance on the particles.

For example, there is used a physical adsorption process in which a protein is brought into direct contact with and immobilized on insoluble carrier particles by means of electrostatic interaction (hydrophobic interaction) between the insoluble carrier particles and the protein. Such a physical adsorption process is suitable for immobilization of a substance having a relatively high molecular weight. However, when such a process is used for immobilizing a low-molecular-weight substance or hapten on insoluble carrier particles, the substance or hapten may be undesirably buried in the surfaces of the insoluble carrier particles, or may be buried by a blocking agent used for preventing non-specific agglutination of the particles. Such a physical adsorption process also encounters difficulty in immobilization of a protein containing a very small amount of hydrophobic amino acid residues.

Alternatively, there is used a chemical bonding process in which, for example, a protein is immobilized on insoluble carrier particles through covalent bonding between a carboxyl group and an amino group at the surface of the insoluble carrier particles or the protein by means of a coupling reagent (e.g. carbodiimide), or through bonding between an amino group of the protein and, for example, an aldehyde group or a tosyl group at the surface of the insoluble carrier particles. Such a chemical bonding process relatively readily achieves immobilization of a low-molecular-weight protein or hapten, which is difficult to achieve by a physical adsorption process. In the case where such a chemical bonding process is used, the functional group of the insoluble carrier can be appropriately selected, to thereby determine a bonding site of the protein responsible for covalent bond formation (i.e. an amino acid residue responsible for covalent bond formation). However, such a chemical bonding process encounters difficulty in immobilization of a protein having a small number of functional groups (i.e. a highly hydrophobic protein). In addition, such a chemical bonding process requires a cumbersome operation for preparation of the immobilizing particles, and may cause denaturation of a protein to be immobilized.

In particular, in the case where a "mixture of a plurality of substances" (e.g. a lysate derived from a microorganism) is immobilized by either a physical adsorption process or a chemical bonding process, biased substances may be immobilized, since each substance has a different size, or in the case of protein, each protein contains a hydrophilic amino acid residue and a hydrophobic amino acid residue in different amounts. Thus, the resultant antigen-immobilizing insoluble carrier particles may encounter difficulty in producing good assay results. Specifically, the particles entrap only a target antibody that binds to limited types of substances, and this may lead to insufficient acquisition of data on, for example, a microorganism that causes a disease from which the target antibody is derived.

Also, a biological body contains a plurality of antibodies against microorganisms; i.e. the biological body contains not a single antibody to a single antigen, but a plurality of antibodies to a plurality of antigens. Thus, entrapment of these antibodies at one time leads to more reliable acquisition of data. As far as the present inventors know, at the time of filing of the present application, there are not provided insoluble carrier particles on which a mixture of many and unspecified substances (e.g., antigenic substances of biological origin) is immobilized as an antigen.

### Means for Solving the Problems

The present inventors have conducted studies for attaining the aforementioned object, and have found that a wide assay range, high sensitivity, and high specificity can be achieved in antibody assay by use of a liquid containing both insoluble carrier particles on which an antigenic substance is immobilized through a physical adsorption process and insoluble carrier particles on which the antigenic substance is immobilized through a chemical bonding process. The present inventors have also found that a wide assay range, high sensitivity, and high specificity can be achieved particularly even when the antigen is a mixture of a plurality of substances (e.g. antigenic substances of biological origin). The present invention has been accomplished on the basis of these findings. The invention is defined by the claims.

In a first aspect, the present invention thus relates to an antibody assay method as defined in claim 1. The antibody assay method comprises the steps of:
bringing a sample collected from a biological body which sample potentially contains a target antibody, into contact with a liquid containing (a) insoluble carrier particles on which a specific antigen is immobilized through physical adsorption (hereinafter the particles may be referred to as the "physical adsorption particles"), and (b) insoluble carrier particles on which the specific antigen is immobilized through chemical bonding (hereinafter the particles may be referred to as the "chemical bonding particles", and the liquid may be referred to as "the antigen-containing liquid of the present invention"); and
detecting agglutination of the insoluble carrier particles caused by antigen-antibody reaction between the antigen immobilized on the insoluble carrier particles and the target antibody contained in the sample,
wherein the specific antigen is a mixture of a plurality of substances and the mixture of a plurality of substances is a lysate derived from a microorganism (hereinafter, the antibody assay method may be referred to as "the assay method of the present invention"). In the antibody assay method of the present invention, a diluent is preferably used in addition to the antigen-containing liquid of the present invention. In the case where a diluent is used, preferably, the sample collected from a biological body and potentially containing a target antibody is diluted with the diluent, and then the diluted sample is brought into contact with the liquid containing the insoluble carrier particles.

In a second aspect, the present invention relates to an antibody assay reagent as defined in claim 4. The antibody assay reagent is a liquid containing insoluble carrier particles, which include both the physical adsorption particles and the chemical bonding particles (i.e. the antigen-containing liquid of the present invention), wherein the specific antigen is a mixture of a plurality of substances and the mixture of a plurality of substances is a lysate derived from a microorganism (hereinafter the antibody assay reagent may be referred to as "the assay reagent of the present invention").

In a third aspect, the present invention relates to an assay kit as defined in claim 7. The assay kit comprises the antibody assay reagent of the second aspect (i.e. the antigen-containing liquid of the present invention) and a diluent (hereinafter the assay kit may be referred to as "the kit of the present invention").

Both the physical adsorption particles and the chemical bonding particles are preferably provided as latex particles. The present invention may involve use of other insoluble carrier particles, such as silica colloid particles, magnetic particles, or metal colloid particles. No particular limitation is imposed on the form of the antigen-containing liquid, so long as the liquid contains insoluble carrier particles. Specifically, the liquid may be in the form of, for example, dispersion, emulsion, suspension, sedimentary liquid, or multiphase liquid. The insoluble carrier particles contained in the antigen-containing liquid may optionally be subjected to surface treatment.

The physical adsorption particles are insoluble carrier particles on which a specific antigen is immobilized by a physical adsorption process as specified above. The physical adsorption particles may have a mean particle size of 0.01 µm to 1.0 µm. The mean particle size is preferably 0.05 µm to 0.35 µm, more preferably 0.10 µm to 0.35 µm, most preferably 0.20 µm to 0.35 µm. The physical adsorption particles may be prepared by bringing an antigen to be adsorbed into contact with a liquid containing insoluble carrier particles, the insoluble carrier particles being a target to which the physical adsorption process is applied.

The chemical bonding particles are insoluble carrier particles on which a specific antigen is immobilized by a chemical bonding process as specified above. The chemical bonding particles may have a mean particle size of 0.01 µm to 1.0 µm. The mean particle size is preferably 0.05 µm to 0.35 µm, more preferably 0.10 µm to 0.35 µm, most preferably 0.20 µm to 0.35 µm. The chemical bonding particles may be prepared by subjecting a liquid containing insoluble carrier particles, the insoluble carrier particles being a target to which the chemical bonding process is applied, to operation for covalent bonding an antigen to be bound.

Preferably, the antigen-containing liquid of the present invention is prepared by mixing the aforementioned "liquid containing physical adsorption particles" and "liquid containing chemical bonding particles".

The mixing ratio (by mass) of the physical adsorption particles and the chemical bonding particles is preferably 10 : 1 to 1 : 10 (physical adsorption particles : chemical bonding particles), more preferably 5 : 1 to 1 : 5 (physical adsorption particles : chemical bonding particles), still more preferably 3 : 1 to 1 : 3 (physical adsorption particles : chemical bonding particles), most preferably 3 : 1 to 1 : 2 (physical adsorption particles : chemical bonding particles). If the amount of the physical adsorption particles is 0.045 mass% or more in the assay reagent used for assay of a target antibody contained in a sample, or in a mixture of the assay reagent and a diluent (when used), improvement in the effect commensurate with an increase in the amount of the particles tends not to be attained.

The term "specific" of "specific antigen" as used herein can be reworded as, for example, "selected". Thus, the specific antigen is "specified" or "selected" as an antigen that binds to a selected antibody to be assayed. Once specified or selected, the antigen is unequivocally determined.

The antigen that can be used as a "specific antigen" can bind to an antibody to be assayed. The antigen may be selected from among various substances, but is preferably an antigen that binds to an antibody contained in a sample collected from a biological body. It is preferable that a substance contained in a biological body in association with a specific disease or constitution is used as a target antibody, and an antigen that binds thereto is used as the "specific antigen". The substance serving as the "specific antigen" is typically a protein, but may be another substance, such as a sugar chain or a lipid.

The term "biological body" as used herein refers to any biological body, and is generally a human body.

Since the antigen is a "mixture of a plurality of substances", the present invention suitably exerts its effects. According to the present invention, the mixture is a lysate derived from a microorganism, such as bacterium or virus.

When the antigen contains at least one substance having a molecular weight of 5,000 or more (particularly preferably 10,000 or more), the present invention suitably exerts its effects. When the physical adsorption particles and the chemical bonding particles are used in combination for a lysate derived from a microorganism, various epitopes can be presented as a whole. No particular limitation is imposed on the maximum of the molecular weight, so long as the antigen can be immobilized on the insoluble carrier particles. The molecular weight is preferably about 2,000,000 or less. The aforementioned "mixture of a plurality of substances" may also apply to the above-mentioned molecular weight conditions.

The aforementioned antigen-containing liquid of the present invention is brought into contact with a sample collected from a biological body and potentially containing a target antibody, to thereby detect the degree of agglutination of the carrier particles caused by antigen-antibody reaction between the antigen immobilized on the insoluble carrier particles contained in the antigen-containing liquid and the target antibody contained in the sample.

No particular limitation is imposed on the "sample collected from a biological body and potentially containing a target antibody", so long as the sample probably contains an antibody to be assayed. Examples of the sample include blood, serum, plasma, urine, lymph fluid, punctate, spinal fluid, sweat, saliva, gastric juice, lung lavage fluid, and feces. Of these, blood, serum, and plasma are preferred.

The "reaction of the specific-antigen-immobilizing insoluble carrier particles caused by antigen-antibody reaction" is mainly agglutination. The agglutination of the insoluble carrier particles can be detected by means of, for example, slide agglutination, optical measurement, microtitration, or filter separation, to thereby assay the antibody of interest.

The assay method of the present invention is preferably performed by means of a biochemical automatic analyzer. The biochemical automatic analyzer is based on the principle of optical measurement. In general, when the analyzer is employed, a sample (e.g. serum) is diluted with a diluent and then heated, followed by addition of a reagent (in the case of the present invention, the antigen-containing liquid of the present invention) to perform assay. Examples of currently available biochemical automatic analyzers include, but are not limited to, HITACHI 7180, LABOSPECT 008, and LABOSPECT 006 (manufactured by Hitachi High-Technologies Corporation); JCA-BM6010, JCA-BM6050, and JCA-BM9130 (manufactured by JEOL Ltd.); TBA-c16000, TBA-2000FR, and TBA-120FR (manufactured by Toshiba Medical Systems Corporation); and AU680 and AU5800 (manufactured by Beckman Coulter, Inc.).

### Effects of the Invention

The present invention provides means for assaying an antibody with a wide assay range and at high sensitivity and high specificity.

### Brief Description of the Drawings

[Fig. 1] A graph showing the results of examination of the effect, on reactivity, of mixing of chemical bonding latex particles having a particle size of 0.145 µm and physical adsorption latex particles having a particle size of 0.235 µm.
[Fig. 2] A graph showing the results of examination of the effect, on reactivity, of the mixing ratio of chemical bonding latex particles having a particle size of 0.300 µm to physical adsorption latex particles having a particle size of 0.235 µm.

### Modes for Carrying Out the Invention

### 1. The antigen-containing liquid of the present invention

As described above, the antigen-containing liquid of the present invention contains both physical adsorption particles and chemical bonding particles.

### (1) Preparation of insoluble carrier particles

As described above, the physical adsorption particles and the chemical bonding particles are preferably provided as latex particles. Latex is an emulsion containing polymer fine particles (latex particles) stably dispersed in an aqueous solvent.

Latex may be prepared through a technique generally employed in the art, such as emulsion polymerization, soap-free emulsion polymerization, seed polymerization, stage feed emulsion polymerization, power feed polymerization, or suspension polymerization. The particle size may be controlled through a technique generally employed in each of these techniques for preparation of latex. In the case where, for example, emulsion polymerization is performed, the particle size of latex can be controlled by regulating, for example, the type and amount of a monomer, an emulsifier, or an initiator, or the polymerization temperature. Needless to say, a commercially available latex product may also be used.

No particular limitation is imposed on the type of latex, so long as it can be applied to a physical adsorption process used for preparation of physical adsorption particles or a chemical bonding process used for preparation of chemical bonding particles. It is preferable to select a latex suitable for the physical adsorption process and a latex suitable for the chemical bonding process.

Examples of the latex suitable for the physical adsorption process include polystyrene latex, ultra-low-content carboxylate latex, and hydrophilic-group-localized latex.

Examples of the latex suitable for the chemical bonding process include latex containing latex particles having on their surfaces, for example, a carboxyl group, a hydroxyl group, an amino group, an aldehyde group, or a tosyl group.

The latex suitable for the physical adsorption process or the latex suitable for the chemical bonding process may be colored latex or fluorescent latex (i.e. latex coated with a fluorescent substance).

As described above, no particular limitation is imposed on the particle size of latex particles used for the physical adsorption process or the chemical bonding process, and the particle size may be 0.01 µm to 1.0 µm. The particle size is preferably 0.05 µm to 0.35 µm, more particularly 0.10 µm to 0.35 µm, most preferably 0.20 µm to 0.35 µm.

The particle size of latex particles used for each of these immobilizing processes may be determined so as to achieve appropriate sensitivity, specificity, and assay range in accordance with the properties of an antigen to be immobilized and an antibody to be assayed. Specifically, latex particles having a medium or large particle size (0.20 to 0.35 µm) are preferably used for increasing the degree of latex agglutination caused by immunoreaction. In the case where, for example, the assay target (antibody) concentration is very high, latex particles having a small particle size (0.10 µm or less) may be preferably used.

As described above, the insoluble carrier particles other than latex particles may be, for example, silica colloid particles, magnetic particles, or metal colloid particles. These insoluble carrier particles may be used in the present invention by subjecting the particles to surface modification (i.e. the particle surfaces are provided with a functional group suitable for physical adsorption or chemical bonding).

### (2) Step of immobilizing antigen on insoluble carrier particles

### (a) Antigen to be immobilized

The antigen to be immobilized on insoluble carrier particlescan bind to a target antibody to be assayed. As described above, since the antigen is a "mixture of a plurality of substances", the present invention suitably exerts its effects. The mixture is a lysate derived from a microorganism, such as bacterium or virus. For example, the *Helicobacter pylori* lysate used in the Examples described hereinbelow contains urease B, urease A, cag A, flagellin, heat shock protein, and other substances. Most of the substances contained in the *Helicobacter pylori* lysate have a molecular weight of about 10,000 to about 200,000.

In order to identify an infecting microorganism in the body (e.g. *Helicobacter pylori*), a substance specific to the infecting microorganism can be used as an antigen to be immobilized. Examples of the microorganism include bacteria, such as *Helicobacter pylori,* group A hemolytic *Streptococcus,* group B hemolytic *Streptococcus,* pneumococcal urinary antigen, *Escherichia coli* 0157, *Clostridium difficile, Legionella, Vibrio cholerae,* and *Meningococcus;* viruses, such as influenza virus, parainfluenza virus, rotavirus, norovirus, Japanese encephalitis virus, rabies virus, poliovirus, echovirus, coxsackie A virus, coxsackie B virus, mumps virus, herpes simplex virus, varicella-zoster virus, measles virus, rubella virus, EB virus, papilloma virus, molluscum contagiosum virus, hand-foot-and-mouth disease virus, acute hemorrhagic conjunctivitis virus, HAV, HBV, HCV, epidemic keratoconjunctivitis virus, hunter virus, human T-lymphotropic virus, HIV, lymphocytic choriomeningitis virus, RS virus, adenovirus, reovirus, rhinovirus, and coronavirus; fungi causing deep mycoses, such as *Candidiasis, Aspergillosis, Cryptococcosis, Zygomycosis, Pneumocystis pneumonia,* and *Trichosporonosis*; rickettsiae causing trombiculiasis, spotted fever, and epidemic typhus; chlamydia; protozoa; and parasite.

Examples of the antigen to be immobilized (other than microorganism-derived antigen) that can be used in the present disclosure (not part of the invention) include antigens against antireceptor antibodies, such as anti-TSH receptor antibody, anti-acetylcholine receptor antibody, and anti-insulin receptor antibody; antigens against antithyroid antibodies, such as antithyroglobulin antibody and antimicrosomal antibody; antigens against anti-pancreatic islet antibodies, such as anti-pancreatic islet cell antibody, anti-GAD antibody, and anti-insulin antibody; and antigens against organ-specific autoantibodies, such as anti-adrenal cortex antibody, anti-smooth muscle antibody, anti-LKM antibody, anti-gastric parietal cell antibody, anti-intrinsic factor antibody, anti-striated muscle antibody, anti-myocardium antibody, anti-skin desmoglein antibody, anti-neutrophil cytoplasm antibody, and anti-phospholipid antibody.

Other examples of the antigen to be immobilized that can be used in the present disclosure (not part of the invention) include antigens against non-organ-specific autoantibodies; for example, autoantigens distributed in chromatin, such as DNA, nucleosome, histone, poly ADP-ribose, centromere, Scl-70, and Ku; antoantigens distributed in nucleoplasm, such as U1RNP, Sm, SS-B/LA, SS-A/Ro, PCNA, and Ki; antoantigens distributed in nucleolus, such as U3RNP, Th/To, PM-Sci, and RNA polymerase; and autoantigens distributed in cytoplasm, such as AA-A/Ro, Jo-1, ribosome P, mitochondria M2, and signal recognition particles.

Currently applicable antigens other than the above-exemplified antigens may be used, and antigens which will be provided in the future may be used (not part of the invention).

### (b) Step of immobilizing antigen

Any of the aforementioned antigens can be immobilized on insoluble carrier particles by a commonly used physical adsorption process or chemical bonding process.

In the physical adsorption process, an antigen to be immobilized can be mixed with, for example, distilled water or PBS containing insoluble carrier particles in an amount of about 0.1 to about 1 mass%, to thereby immobilize the antigen on the insoluble carrier particles. Subsequently, non-adsorbed antigen and antigen-immobilizing carrier particles can be separated by means of centrifugation, and the particles are redispersed in an aqueous solvent (generally a buffer), to thereby prepare a liquid containing physical adsorption particles. Examples of the buffer include Good's buffer (e.g. HEPES buffer), TRIS buffer, glycine buffer, and borate buffer.

In the chemical bonding process, a specific antigen is covalently bonded to the functional group on the surfaces of insoluble carrier particles. In the case where, for example, the insoluble carrier particles have on the surfaces thereof a carboxyl group, a hydroxyl group, or an amino group, carbodiimide, bromcyan, or glutaraldehyde can be respectively used as an activator so that the antigen is covalently bonded to the surfaces of the insoluble carrier particles. The functional group on the surfaces of the insoluble carrier particles may be activated by means of the activator before the antigen is brought into contact with the particles. Alternatively, the activator and the antigen may be simultaneously brought into contact with the insoluble carrier particles. When the functional group on the surfaces of the insoluble carrier particles is an aldehyde group or a tosyl group, the activator does not need to be used. The covalent bonding may be performed by introducing a spacer molecule (e.g. oligoamino acid or aminocarboxylic acid) between the insoluble carrier particles and the antigen.

The aforementioned chemical bonding reaction is performed in an aqueous solvent (generally a buffer). The resultant chemical bonding particles can be redispersed in an aqueous solvent (generally a buffer), to thereby prepare a liquid containing chemical bonding particles. Examples of the buffer include Good's buffer (e.g. HEPES buffer), TRIS buffer, glycine buffer, and borate buffer.

Thus, the physical adsorption particles and the chemical bonding particles can be prepared. The physical adsorption particles and the chemical bonding particles are stored in an aqueous solvent in the form of a liquid containing insoluble carrier particles.

### (3) The antigen-containing liquid of the present invention

The antigen-containing liquid of the present invention contains both the physical adsorption particles and the chemical bonding particles, and the solvent used for the liquid is generally a buffer. Examples of the buffer include Good's buffer (e.g. HEPES buffer), TRIS buffer, glycine buffer, and borate buffer. For example, the antigen-containing liquid of the present invention can be prepared by mixing the above-prepared physical adsorption particles (in the form of physical adsorption particles-containing liquid) with the above-prepared chemical bonding particles (in the form of chemical bonding particles-containing liquid) in an aqueous solvent (generally a buffer). The method for preparing the antigen-containing liquid of the present invention is not limited thereto, so long as a liquid containing both the physical adsorption particles and the chemical bonding particles can be prepared.

The antigen-containing liquid of the present invention may optionally contain, for example, an electrolyte, a stabilizer, a surfactant, or a sensitizer.

In the antigen-containing liquid of the present invention, the proportions of both types of antigen-immobilizing carrier particles may vary depending on the desired assay range or sensitivity. The proportions (by mass) are preferably 10 : 1 to 1 : 10 (physical adsorption particles : chemical bonding particles), more preferably 5 : 1 to 1 : 5 (physical adsorption particles : chemical bonding particles), still more preferably 3 : 1 to 1 : 3 (physical adsorption particles : chemical bonding particles), most preferably 3 : 1 to 1 : 2 (physical adsorption particles : chemical bonding particles). If the amount of the physical adsorption particles is 0.045 mass% or more in the antigen-containing liquid used for assay of a target antibody contained in a sample, or in a mixture of the antigen-containing liquid and a diluent (when used), improvement in the effect commensurate with an increase in the amount of the particles tends not to be attained.

### 2. Antibody assay

The assay method of the present invention involves assay of an antibody by use of the aforementioned antigen-containing liquid of the present invention. Specifically, the antigen-containing liquid of the present invention is brought into contact with a sample collected from a biological body and potentially containing a target antibody, to thereby detect the reaction of the fine particles caused by antigen-antibody reaction between the antigen immobilized on the insoluble carrier particles contained in the antigen-containing liquid and the target antibody contained in the sample. The assay method of the present invention may be performed without use of a diluent. Preferably, the assay method is performed by use of a diluent.

In the case where the assay method is performed by use of a diluent, the antigen-containing liquid of the present invention may be mixed and diluted with the diluent, and then may be brought into contact with the sample. Preferably, the sample is diluted with the diluent, and the diluted sample is brought into contact with the antigen-containing liquid of the present invention. The diluent is preferably used in an amount by volume 9 times or less (particularly preferably one to five times) that of the antigen-containing liquid of the present invention.

As described above, no particular limitation is imposed on the "sample collected from a biological body and potentially containing a target antibody", so long as the sample probably contains an antibody to be assayed. Examples of the sample include blood, serum, plasma, urine, lymph fluid, punctate, spinal fluid, sweat, saliva, gastric juice, lung lavage fluid, and feces. Of these, blood, serum, and plasma are preferred. The sample (e.g. serum) may optionally be diluted with, for example, saline. The "sample collected from a biological body and potentially containing a target antibody" includes the sample diluted with, for example, saline. This dilution differs from the aforementioned dilution with a diluent.

### 3. The kit of the present invention

The assay kit of the present invention is used for performing the assay method of the present invention. The kit contains, as components, the antigen-containing liquid of the present invention and a diluent described below.

The component (other than the antigen-containing liquid of the present invention) of the kit of the present invention is, for example, a standard product of a target antibody to be assayed, a sample for accuracy control, or a diluent.

The diluent is preferably a liquid used for diluting the "sample collected from a biological body and potentially containing a target antibody" before the sample is brought into contact with the antigen-containing liquid of the present invention. No particular limitation is imposed on the diluent, so long as it causes substantially no inhibition of the assay of the target antibody. As in the case of the antigen-containing liquid of the present invention, the diluent is typically a buffer. Examples of the buffer include Good's buffer (e.g. HEPES buffer), TRIS buffer, glycine buffer, and borate buffer. The diluent may optionally contain, for example, an electrolyte, a stabilizer, a surfactant, or a sensitizer. The diluent is preferably identical or similar to the solvent used in the antigen-containing liquid of the present invention.

In the case where the assay method of the present invention is performed by use of the kit of the present invention, the assay may be performed without use of the diluent. When the diluent is used, the amount by volume of the diluent is preferably 9 times or less (particularly preferably one to five times) that of the antigen-containing liquid of the present invention.

In the Examples described below, a first reagent (diluent upon use) is the aforementioned diluent, and a second reagent (latex dispersion) is the antigen-containing liquid of the present invention. One specific example of the kit of the present invention contains the first and second reagents as components.

### Examples

The present invention will next be described by way of example. Unless otherwise specified, the symbol "%" refers to mass%.

### [Production Example]

### 1. Latex

Latexes used in the Examples are as follows.
(1) Latexes used for a physical adsorption process
   0.235 µm polystyrene latex particles (hereinafter referred to as "physical latex 1")
   0.223 µm polystyrene latex particles (hereinafter referred to as "physical latex 2")
   0.310 µm polystyrene latex particles (hereinafter referred to as "physical latex 3")
(2) Latexes used for a chemical bonding process
   0.145 µm carboxyl latex particles (hereinafter referred to as "chemical latex 1")
   0.245 µm carboxyl latex particles (hereinafter referred to as "chemical latex 2")
   0.300 µm carboxyl latex particles (hereinafter referred to as "chemical latex 3")

### 2. Preparation of antigen to be immobilized

In the Examples, an antigen prepared from *Helicobacter pylori* was used as a bacterium-derived antigen to be immobilized. The bacterium-derived antigen was prepared through the procedure described below.

### (1) Preliminary culture

The original bacterial cells were cultured at 37°C under a microaerophilic environment (prepared by Anaero Pack (Mitsubishi Gas Chemical Company, Inc.)) by use of Pourmedia (registered trademark) for selection/separation of *Helicobacter pylori* (HP separation medium) (Eiken Chemical Co., Ltd.) .

### (2) Main culture

The culture medium used for main culture was a medium (200 mL) prepared by addition of 5% horse serum to Pearlcore (registered trademark) brain heart infusion broth "Eiken" (Eiken Chemical, Co., Ltd.). A single colony on the aforementioned preliminary culture medium was fished and adjusted to McFarland No. 1.0 to obtain an inoculum solution. The inoculum solution was inoculated into the main culture medium. Thereafter, shaking culture was performed under a microaerophilic gas atmosphere at 37°C.

### (3) Bacteriolysis

After completion of the aforementioned main culture, the bacterial cells were collected by centrifugation, and then washed by centrifugation with saline. The resultant precipitate was mixed with a lysis solution (50mM HEPES buffer containing 0.15M NaCl, 0.1% polyoxyethylene octyl phenyl ether, 5mM EDTA, and 0.1% NaN₃, pH 7.4), and the resultant mixture was subjected to bacteriolysis by freeze-thawing .

### (4) Antigen preparation

The resultant lysate of *Helicobacter pylori* was dialyzed against HEPES buffer (pH 7.4) containing 0.15M NaCl and 0.1% NaN₃, followed by centrifugation. The resultant supernatant was filtered with a membrane filter having a pore size of 0.45 µm. The filtrate was provided as an antigen (i.e. completion of the antigen preparation), and the antigen was used as "*Helicobacter pylori* lysate" in the subsequent step. The protein concentration of *Helicobacter pylori* lysate (antigen) was measured by the BCA method.

### 3. Immobilization step on latex particles

### (1) Immobilization by physical adsorption process

0.5M CHES buffer (pH 8.8) (0.48 mL) was added to a 0.09% polystyrene latex particle-containing liquid (21.1 mL), and the mixture was mixed with 8-mg/mL *Helicobacter pylori* lysate (0.25 mL). The resultant mixture was agitated at room temperature for 30 minutes, and then 10% BSA solution (1.04 mL) was added to the mixture. Purified water was added to the mixture to achieve a total amount of 24.0 mL. Subsequently, the mixture was heated at 56°C for four hours, and then subjected to centrifugation, to thereby remove non-adsorbed antigen. The latex particles obtained through removal of non-adsorbed antigen by centrifugation was redispersed in 0.01M HEPES buffer (pH 7.4) (4.0 mL), and 10% BSA solution (0.04 mL) was added to the dispersion. Thereafter, the resultant mixture was heated at 56°C for two hours. The thus-heated liquid containing antigen-adsorbed latex was subjected to filtration, to thereby prepare a physical adsorption latex having a latex particle concentration of 0.48%.

The amount of 0.01M HEPES buffer (pH 7.4) used for redispersion of the latex particles after removal of non-adsorbed antigen by centrifugation may be appropriately modified for appropriate adjustment of the latex particle concentration of the resultant physical adsorption latex (the same shall apply to a chemical bonding process).

### (2) Immobilization by chemical bonding process

1-Ethyl-3-(3-dimethylaminopropyl)carbodiimide hydrochloride (2 equivalents by mole of a carboxyl group) was added to a 0.3% carboxyl latex particle-containing liquid (6.4 mL), and the mixture was agitated for 15 minutes, to thereby activate the carboxyl group. Purified water (63.2 mL) and 0.5M HEPES buffer (pH 7.0) (1.28 mL) were added to the liquid containing the carboxyl-activated latex particles, and the mixture was mixed with 8-mg/mL *Helicobacter pylori* lysate (0.340 mL), followed by reaction for three hours. The resultant reaction mixture was mixed with 10% BSA solution (0.776 mL), followed by reaction for four hours. The resultant mixture was subjected to centrifugation, to thereby remove non-adsorbed antigen. The latex particles obtained through removal of non-bonded antigen by centrifugation was redispersed in 0.01M HEPES buffer (pH 7.4) (4.0 mL), and 10% BSA solution (0.04 mL) was added to the dispersion. Thereafter, the resultant mixture was heated at 56°C for four hours. The thus-heated liquid containing antigen-bonded latex was subjected to filtration, to thereby prepare a chemical bonding latex having a latex particle concentration of 0.48%.

### (3) Preparation of latex particle dispersion

The physical adsorption latex (1.563 mL) prepared in (1) above and the chemical bonding latex (0.521 mL) prepared in (2) above were added to HEPES buffer (pH 7.4) (2.5 mL) containing 0.3M NaCl, 0.8M L-arginine hydrochloride, and 0.2% NaN₃, and purified water was added to the mixture to achieve a total amount of 5.0 mL, to thereby prepare a latex particle dispersion containing 0.15% physical adsorption latex particles and 0.05% chemical bonding latex particles.

The mixing proportion can be modified by appropriately determining the amounts of the physical adsorption latex and the chemical bonding latex to be mixed.

### 4. Assay system

The assay in the Examples was performed through the turbidimetric method.

The first reagent was a diluent (diluent upon use) containing 0.05M HEPES, 0.15M NaCl, 0.5% chondroitin sulfate Na, 0.1% BSA, 0.1% PBC-34, 0.1% NaN₃, and purified water (balance) (pH 7.4).

The second reagent was the latex particle dispersion (the antigen-containing liquid of the present invention) containing 0.05M HEPES, 0.15M NaCl, 0.4M L-Arg-HCl, 0.1% NaN₃, latex particles, and purified water (balance) (pH 7.4).

The assay was performed by means of a biochemical automatic analyzer JCA-BM2250 (JEOL Ltd.).

Specifically, in the analyzer, a sample (serum sample) was 5-fold diluted with saline, the diluted sample (6.0 µL) was mixed with the first reagent (60 µL), and the mixture was incubated at 37°C for 5 minutes. Thereafter, the mixture was mixed with the second reagent (20 µL), followed by reaction at 37°C. A change in absorbance for about three minutes after mixing with the second reagent was measured at two wavelengths: 658 nm (main wavelength) and 805 nm (sub-wavelength). In the Examples, the amount by volume of the first reagent was three times that of the second reagent (i.e. 4-fold dilution).

The ELISA method (E Plate "Eiken" H.pylori II: Eiken Chemical Co., Ltd.) was used as a reference method.

### [Example 1] Examination of the effect of mixing of two latex particles

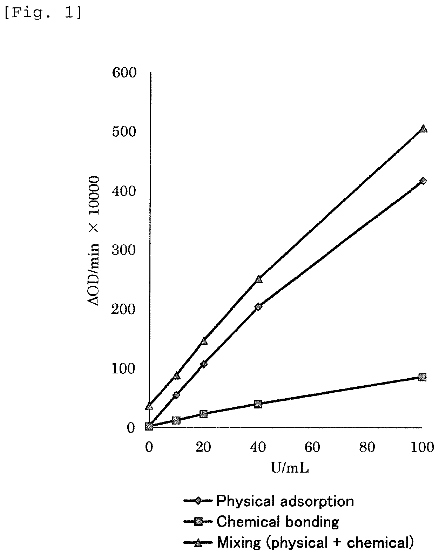

There were used two latex particles on which the *Helicobacter pylori* lysate was immobilized by different processes as described above; i.e. physical adsorption latex particles having a particle size of 0.235 µm (physical latex 1) and chemical bonding latex particles having a particle size of 0.145 µm (chemical latex 1). A dilution series of pooled serum was used to examine the reactivity in the following three cases: the case where only the physical adsorption latex particles were used; the case where only the chemical bonding latex particles were used; and the case where a mixture of these latex particles was used (Table 1 and Fig. 1). The latex particle concentration in the second reagent used for examination was adjusted as follows: 0.15% in the case where only the physical adsorption latex particles were used; 0.30% in the case where only the chemical bonding latex particles were used; and 0.15% (the physical adsorption latex particles) and 0.30% (the chemical bonding latex particles) in the case where the mixture was used. Subsequently, the serum sample was assayed for determining the specificity for each of the aforementioned cases (Table 2). The "unit of reactivity in Table 1" and the "unit of the vertical axis in Fig. 1" correspond to a change in absorbance (ΔOD/min) multiplied by 10,000.

As described above, the concentrations of the physical adsorption latex particles and chemical bonding latex particles shown in Table 1 are the concentrations in the second reagent, and the concentrations thereof upon assay is 1/4 of that shown in Table 1 (because of dilution with the first reagent). For example, "physical latex 1 0.15%" shown in Table 1 corresponds to the concentration of the physical latex 1 upon assay of 0.0375% (i.e. 0.15 × 1/4 = 0.0375%).

**[Table 1]**

| std | U/mL | Physical adsorption physical latex 1 0.15% ΔOD/min × 10000 | Chemical bonding chemical latex 1 0.30% ΔOD/min × 10000 | Mixing (physical + chemical) 0.15% + 0.30% ΔOD/min × 10000 |
|---|---|---|---|---|
| S1 | 0 | 2 | 2 | 37 |
| 52 | 10 | 55 | 12 | 89 |
| S3 | 20 | 107 | 23 | 147 |
| S4 | 40 | 204 | 40 | 251 |
| 55 | 100 | 417 | 86 | 506 |

**[Table 2]**

| | | Physical adsorption | | | Chemical bonding | | | Mixing (physical + chemical) | | |
|---|---|---|---|---|---|---|---|---|---|---|
| | | Positive | Negative | Concordance rate | Positive | Negative | Concordance rate | Positive | Negative | Concordance rate |
| ELISA | Positive | 71 | 27 | 72% | 83 | 15 | 85% | 83 | 15 | 85% |
| | Negative | 13 | 293 | 96% | 78 | 228 | 75% | 13 | 293 | 96% |
| | Total | 84 | 320 | 90% | 161 | 243 | 77% | 96 | 308 | 93% |

Mixing of the physical adsorption latex particles with the chemical bonding latex particles resulted in maintenance of high negative concordance rate and an increase in positive concordance rate (i.e. an increase in specificity). Favorable results were obtained in terms of overall concordance rate when the concentrations of the physical adsorption latex particles and the chemical bonding latex particles in the second reagent were respectively 0.15% and 0.30% (1 : 2) (Table 2).

### [Example 2] Examination of mixing proportions of two latex particles

With using latexes on which the *Helicobacter pylori* lysate was immobilized through different processes as described above, the concentration of the physical adsorption latex particles having a particle size of 0.223 µm (physical latex 2) in the second reagent was adjusted to 0.20%, and then the concentration of the chemical bonding latex particles having a particle size of 0.145 µm (chemical latex 1) was adjusted to 0 to 0.20%. The serum sample was assayed by use of the resultant second reagent for determining the specificity (Table 3).

The relationship between the concentrations of the physical adsorption latex particles and the chemical bonding latex particles shown in Table 3 and the concentrations thereof at dilution (4-fold) with the first reagent upon assay is as described in Example 1.

**[Table 3]**

| Physical adsorption latex = 0.223 µm, Chemical bonding latex = 0.145 µm | | | | | | | | | | | | | |
|---|---|---|---|---|---|---|---|---|---|---|---|---|---|
| | Mixing proportions | Physical = 0.20% | | | Physical = 0.20% : Chemical = 0.05% | | | Physical = 0.20% : Chemical = 0.10% | | | Physical = 0.20% : Chemical = 0.20% | | |
| | | Positive | Negative | Concordance rate | Positive | Negative | Concordance rate | Positive | Negative | Concordance rate | Positive | Negative | Concordance rate |
| ELISA | Positive | 8 | 7 | 53% | 10 | 5 | 67% | 11 | 4 | 73% | 12 | 3 | 80% |
| | Negative | 1 | 5 | 83% | 1 | 5 | 83% | 2 | 4 | 67% | 2 | 4 | 67% |
| | Total | 9 | 12 | 62% | 11 | 10 | 71% | 13 | 8 | 71% | 14 | 7 | 76% |

In the case of mixing of the chemical bonding latex particles, an increase in the amount of the mixed chemical bonding latex particles led to an increase in concordance rate. In particular, as the concentration of the chemical bonding latex particles in the second reagent increased from 0% to 0.05%, 0.10%, and 0.20%, the positive concordance rate markedly improved from 53%, to 67%, 73%, and 80%. This shows that false-negative (missed) can be avoided. However, since the amount of the mixed physical adsorption latex particles was 0.20%; i.e. greater than the below-described amount (0.18%, corresponding to 0.045% upon assay), an increase in concordance rate was suppressed (Table 3).

### [Example 3] Examination of mixing proportions of two latex particles (1)

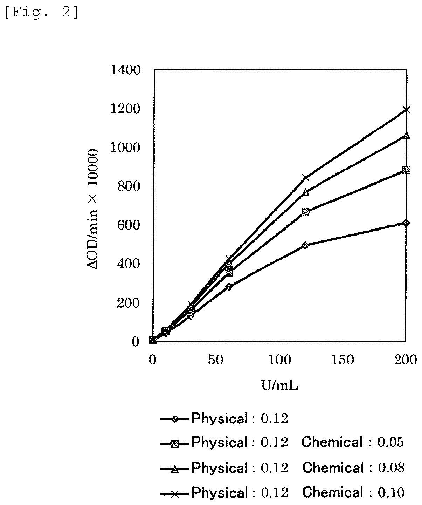

With using latex particles on which the *Helicobacter pylori* lysate was immobilized through different processes as described above, the concentration of the physical adsorption latex particles having a particle size of 0.235 µm (physical latex 1) in the second reagent was adjusted to 0.12%, and then the concentration of the chemical bonding latex particles having a particle size of 0.300 µm (chemical latex 3) was adjusted to 0 to 0.10%. The reactivity was examined by means of LZ H.pylori antibody calibrator "Eiken" (Eiken Chemical Co., Ltd.) (Table 4, Fig. 2). Subsequently, the serum sample was assayed by use of the second reagent for determining the specificity (Table 5). The "unit of reactivity in Table 4" and the "unit of the vertical axis in Fig. 2" correspond to a change in absorbance (ΔOD/min) multiplied by 10,000.

The relationship between the concentrations of the physical adsorption latex particles and the chemical bonding latex particles shown in Tables 4 and 5 and the concentrations thereof at dilution (4-fold) with the first reagent upon assay is as described in Example 1.

**[Table 4]**

| | | | | | |
|---|---|---|---|---|---|
| Physical adsorption | Physical latex 1 (%) | 0.12 | 0.12 | 0.12 | 0.12 |
| Chemical bonding | Chemical latex 3 (%) | | 0.05 | 0.08 | 0.10 |
| std | U/mL | ΔOD/min × 10000 | ΔOD/min × 10000 | ΔOD/min × 10000 | ΔOD/min × 10000 |
| S1 | 0 | 6 | 10 | 10 | 10 |
| 52 | 10 | 42 | 52 | 58 | 57 |
| S3 | 30 | 132 | 164 | 180 | 192 |
| S4 | 60 | 283 | 354 | 400 | 424 |
| 55 | 120 | 494 | 665 | 769 | 842 |
| 56 | 200 | 610 | 881 | 1061 | 1194 |

**[Table 5]**

| Physical adsorption latex = 0.223 µm, Chemical bonding latex = 0.300 µm | | | | | | | | | | | | | |
|---|---|---|---|---|---|---|---|---|---|---|---|---|---|
| | Mixing proportions | Physical = 0.12% | | | Physical = 0.12% : Chemical = 0.05% | | | Physical = 0.12% : Chemical = 0.08% | | | Physical = 0.12% : Chemical = 0.10% | | |
| | | Positive | Negative | Concordance rate | Positive | Negative | Concordance rate | Positive | Negative | Concordance rate | Positive | Negative | Concordance rate |
| ELISA | Positive | 63 | 18 | 78% | 67 | 14 | 83% | 74 | 7 | 91% | 76 | 5 | 94% |
| | Negative | 3 | 156 | 98% | 3 | 156 | 98% | 10 | 149 | 94% | 11 | 148 | 93% |
| | Total | 66 | 174 | 91% | 70 | 170 | 93% | 84 | 156 | 93% | 87 | 153 | 93% |

As shown by the results, an increase in the mixing ratio of the chemical bonding latex particles to the physical adsorption latex particles led to an increase in positive concordance rate, and false-negative (missed) could be avoided. Furthermore, an increase in the amount of the mixed chemical bonding latex particles led to an increase in reactivity. When the concentration ratio of the physical adsorption latex particles and the chemical bonding latex particles was 12 : 5 to 12 : 10 (within a range of 3 : 1 to 1 : 3) in the second reagent, high overall concordance rate was achieved (Table 5).

### [Example 4] Examination of mixing proportions of two latex particles (2)

An examination was performed with the same purpose as in Example 3 in a wider assay range. As in Example 3, physical latex 1 and chemical latex 3 were used as latex particles.
(1) The concentration of the physical latex 1 in the second reagent was fixed to 0.12%, and the concentration of the chemical latex 3 was adjusted to 0% (physical adsorption : chemical bonding = 7.5 : 0), 0.016% (7.5 : 1), 0.048% (7.5 : 3), 0.08% (7.5 : 5), 0.12% (7.5 : 7.5), or 0.16% (7.5 : 10). In each case, the serum sample was assayed for determining the specificity (Table 6). *H. pylori* antibody level was determined on the basis of a calibration curve prepared by means of LZ H.pylori antibody calibrator "Eiken" (Eiken Chemical Co., Ltd.).

The relationship between the concentrations of the physical adsorption latex particles or the chemical bonding latex particles shown in Table 6 and the concentrations thereof at dilution (4-fold) with the first reagent upon assay is as described in Example 1.

**[Table 6]**

| | | Phys. = 0.12% : Chem. = None | | | Phys. = 0.12% : Chem. 0.016% | | | Phys. = 0.12% : Chem. 0.048% | | | Phys. = 0.12% : Chem. 0.08% | | | Phys. = 0.12% : Chem. 0.12% | | | Phys. = 0.12% : Chem. 0.16% | | |
|---|---|---|---|---|---|---|---|---|---|---|---|---|---|---|---|---|---|---|---|
| | | Positive | Negative | Concordance rate | Positive | Negative | Concordance rate | Positive | Negative | Concordance rate | Positive | Negative | Concordance rate | Positive | Negative | Concordance rate | Positive | Negative | Concordance rate |
| ELISA | Positive | 9 | 4 | 69% | 10 | 3 | 77% | 11 | 2 | 85% | 13 | 0 | 100% | 12 | 1 | 92% | 12 | 1 | 92% |
| | Negative | 0 | 5 | 100% | 0 | 5 | 100% | 0 | 5 | 100% | 0 | 5 | 100% | 1 | 4 | 80% | 1 | 4 | 80% |
| | Total | 9 | 9 | 78% | 10 | 8 | 83% | 11 | 7 | 89% | 13 | 5 | 100% | 13 | 5 | 89% | 13 | 5 | 89% |

(2) The concentration of the chemical latex 3 in the second reagent was fixed to 0.08%, and the concentration of the physical latex 1 was adjusted to 0% (physical adsorption : chemical bonding = 0 : 5), 0.024% (1.5 : 5), 0.072% (4.5 : 5), 0.12% (7.5 : 5), 0.180 (11.3 : 5), or 0.24% (15 : 5). In each case, the serum sample was assayed for determining the specificity (Table 7). *H. pylori* antibody level was determined on the basis of a calibration curve prepared by means of LZ H.pylori antibody calibrator "Eiken" (Eiken Chemical Co., Ltd.).

The relationship between the concentrations of the physical adsorption latex particles and the chemical bonding latex particles shown in Table 7 and the concentrations thereof at dilution (4-fold) with the first reagent upon assay is as described in Example 1.

**[Table 7]**

| | | Phys. = None : Chem. 0.08% | | | Phys. = 0.024% : Chem. 0.08% | | | Phys. = 0.072% : Chem. 0.08% | | | Phys. = 0.12% : Chem. 0.08% | | | Phys. = 0.18% : Chem. 0.08% | | | Phys. = 0.24% : Chem. 0.08% | | |
|---|---|---|---|---|---|---|---|---|---|---|---|---|---|---|---|---|---|---|---|
| | | Positive | Negative | Concordance rate | Positive | Negative | Concordance rate | Positive | Negative | Concordance rate | Positive | Negative | Concordance rate | Positive | Negative | Concordance rate | Positive | Negative | Concord ance rate |
| ELISA | Positive | 13 | 0 | 100% | 12 | 1 | 92% | 13 | 0 | 100% | 13 | 0 | 100% | 10 | 3 | 77% | 10 | 3 | 77% |
| | Negative | 2 | 3 | 60% | 0 | 5 | 100% | 0 | 5 | 100% | 0 | 5 | 100% | 1 | 4 | 80% | 1 | 4 | 80% |
| | Total | 15 | 3 | 89% | 12 | 6 | 94% | 13 | 5 | 100% | 13 | 5 | 100% | 11 | 7 | 78% | 11 | 7 | 78% |

In (1) and (2) above, the effect of mixing of the physical adsorption latex particles and the chemical bonding latex particles was observed in a range wider than that in the case of Example 3 described above.

Specifically, when the concentration ratio between the physical adsorption latex particles and the chemical bonding latex particles was adjusted to fall within a range of 7.5 : 1 to 7.5 : 10 in the second reagent, the effect of mixing of these two latex particles was observed in the case of 7.5 : 1. When the concentration ratio between these two latex particles was 7.5 : 3 to 7.5 : 10 (within a range of 3 : 1 to 1 : 3), high overall concordance rate was achieved (Table 6).

When the above-mentioned concentration ratio was adjusted to fall within a range of 1.5 : 5 to 15 : 5, high overall concordance rate was achieved in the cases of 1.5 : 5 (within a range of 5 : 1 to 1 : 5), and, 4.5 : 5 and 7.5 : 5 (within a range of 3 : 1 to 1 : 3). However, in the case where the concentration of the physical adsorption latex particles in the second reagent was 0.18% (0.045% upon assay) or more (i.e. in the two cases of 11.3 : 5 and 15 : 5), an improvement in specificity attained by latex mixing was prevented despite the mixing ratio falling within a range of 3 : 1 to 1 : 3 (Table 7).

In the case where either the physical adsorption latex particles or the chemical bonding latex particles were used, a very large difference was observed between positive concordance rate and negative concordance rate. This is probably attributed to a small number of samples.

### [Example 5] Examination of the particle size of latex particles

With using latex particles on which the *Helicobacter pylori* lysate was immobilized through different processes as described above, the concentration of the physical adsorption latex particles having a particle size of 0.235 µm (physical latex 1) in the second reagent was adjusted to 0.15%, and then the concentration of the chemical bonding latex particles having a particle size of 0.245 µm or 0.300 µm (chemical latex 2 (medium particle size) or chemical latex 3 (large particle size)) was adjusted to 0.05%. In each case, the serum sample was assayed for determining the specificity. *H. pylori* antibody level was determined on the basis of a calibration curve prepared by means of LZ H.pylori antibody calibrator "Eiken" (Eiken Chemical Co., Ltd.). As a result, the overall concordance rate (positive concordance rate + negative concordance rate) was 92% or 88%, respectively, and no substantial difference was observed between these cases.

In addition, in order to determine the effect of the particle size of the physical adsorption latex particles on assay values, physical latex 2 (particle size: 0.223 µm (medium particle size)) and physical latex 3 (particle size: 0.310 µm (large particle size)) were used, and the concentration of each physical latex in the second reagent was adjusted to 0.10%. In each case, the specificity was determined in the same manner as described above. Substantially no difference was observed in positive concordance rate, negative concordance rate, and overall concordance rate between these cases.

In Example 5, the relationship between the concentrations of the physical adsorption latex particles and the chemical bonding latex particles and the concentrations thereof at dilution (4-fold) with the first reagent upon assay is as described in Example 1.

From the above results, the particle size (at least medium or large particle size) of the chemical bonding latex particles or the physical adsorption latex particles was found to have substantially no effect on the specificity of assay of a sample collected from a biological body.

## Claims

1. An antibody assay method, the method comprising the steps of:
bringing a sample collected from a biological body and potentially containing a target antibody into contact with a liquid containing (a) insoluble carrier particles on which a specific antigen is immobilized by physical adsorption, and (b) insoluble carrier particles on which the specific antigen is immobilized by covalent bonding; and
detecting agglutination of the insoluble carrier particles caused by antigen-antibody reaction between the antigen immobilized on the insoluble carrier particles and the target antibody contained in the sample,
wherein the specific antigen is a mixture of a plurality of substances and the mixture of a plurality of substances is a lysate derived from a microorganism.

2. The antibody assay method according to claim 1, wherein the specific antigen contains at least one substance having a molecular weight of 5,000 or more.

3. The antibody assay method according to claim 1 or 2, wherein the insoluble carrier particles are latex particles.

4. An antibody assay reagent for detecting reaction of antigen-immobilizing carrier particles caused by antigen-antibody reaction with a target antibody contained in a sample collected from a biological body,
wherein the antibody assay reagent is a liquid containing (a) insoluble carrier particles on which a specific antigen is immobilized by physical adsorption, and (b) insoluble carrier particles on which the specific antigen is immobilized by covalent bonding, and
wherein the specific antigen is a mixture of a plurality of substances and the mixture of a plurality of substances is a lysate derived from a microorganism.

5. The antibody assay reagent according to claim 4, wherein the specific antigen contains at least one substance having a molecular weight of 5,000 or more.

6. The antibody assay reagent according to claim 4 or 5, wherein the insoluble carrier particles are latex particles.

7. An assay kit comprising:
the antibody assay reagent according to any one of claims 4 to 6; and
a diluent.

## Patentansprüche

1. Antikörper-Assay-Verfahren, wobei das Verfahren die Schritte umfasst:
Inkontaktbringen einer Probe, die von einem biologischen Körper gesammelt wurde und möglicherweise einen Zielantikörper enthält, mit einer Flüssigkeit, welche (a) unlösliche Trägerpartikel, auf denen ein spezifisches Antigen durch physikalische Adsorption immobilisiert ist, und (b) unlösliche Trägerpartikel, auf denen das spezifische Antigen durch kovalente Bindung immobilisiert ist, enthält; und
Nachweisen einer Agglutination der unlöslichen Trägerpartikel, welche durch eine Antigen-Antikörper-Reaktion zwischen dem auf den unlöslichen Trägerpartikeln immobilisierten Antigen und dem in der Probe enthaltenen Zielantikörper hervorgerufen wird,
wobei es sich bei dem spezifischen Antigen um ein Gemisch aus einer Vielzahl von Substanzen handelt und es sich bei dem Gemisch aus einer Vielzahl von Substanzen um ein aus einem Mikroorganismus erhaltenes Lysat handelt.

2. Antikörper-Assay-Verfahren gemäß Anspruch 1, wobei das spezifische Antigen zumindest eine Substanz mit einem Molekulargewicht von 5000 oder mehr enthält.

3. Antikörper-Assay-Verfahren gemäß Anspruch 1 oder 2, wobei es sich bei den unlöslichen Trägerpartikeln um Latexpartikel handelt.

4. Antikörper-Assay-Reagenz zum Nachweis der Reaktion von Antigen-immobilisierenden Trägerpartikeln, welche durch eine Antigen-Antikörper-Reaktion mit einem Zielantikörper, der in einer von einem biologischen Körper gesammelten Probe enthalten ist, hervorgerufen wird,
wobei es sich bei dem Antikörper-Assay-Reagenz um eine Flüssigkeit handelt, welche (a) unlösliche Trägerpartikel, auf denen ein spezifisches Antigen durch physikalische Adsorption immobilisiert ist, und (b) unlösliche Trägerpartikel, auf denen das spezifische Antigen durch kovalente Bindung immobilisiert ist, enthält, und
wobei es sich bei dem spezifischen Antigen um ein Gemisch aus einer Vielzahl von Substanzen handelt und es sich bei dem Gemisch aus einer Vielzahl von Substanzen um ein aus einem Mikroorganismus erhaltenes Lysat handelt.

5. Antikörper-Assay-Reagenz gemäß Anspruch 4, wobei das spezifische Antigen zumindest eine Substanz mit einem Molekulargewicht von 5000 oder mehr enthält.

6. Antikörper-Assay-Reagenz gemäß Anspruch 4 oder 5, wobei es sich bei den unlöslichen Trägerpartikeln um Latexpartikel handelt.

7. Assay-Kit, umfassend:
das Antikörper-Assay-Reagenz gemäß einem der Ansprüche 4 bis 6; und
ein Verdünnungsmittel.

## Revendications

1. Procédé de dosage d'anticorps, le procédé comprenant les étapes :
d'amenée d'un échantillon recueilli à partir d'un corps biologique et contenant potentiellement un anticorps cible en contact avec un liquide contenant (a) des particules porteuses insolubles sur lesquelles un antigène spécifique est immobilisé par adsorption physique, et (b) des particules porteuses insolubles sur lesquelles l'antigène spécifique est immobilisé par liaison covalente ; et
de détection d'agglutination des particules porteuses insolubles provoquée par une réaction antigène-anticorps entre l'antigène immobilisé sur les particules porteuses insolubles et l'anticorps cible contenu dans l'échantillon,
dans lequel l'antigène spécifique est un mélange d'une pluralité de substances et le mélange d'une pluralité de substances est un lysat dérivé d'un micro-organisme.

2. Procédé de dosage d'anticorps selon la revendication 1, dans lequel l'antigène spécifique contient au moins une substance ayant un poids moléculaire de 5000 ou plus.

3. Procédé de dosage d'anticorps selon la revendication 1 ou 2, dans lequel les particules porteuses insolubles sont des particules de latex.

4. Réactif de dosage d'anticorps pour détecter une réaction de particules porteuses d'immobilisation d'antigène provoquée par une réaction antigène-anticorps avec un anticorps cible contenu dans un échantillon recueilli à partir d'un corps biologique,
dans lequel le réactif de dosage d'anticorps est un liquide contenant (a) des particules porteuses insolubles sur lesquelles un antigène spécifique est immobilisé par adsorption physique, et (b) des particules porteuses insolubles sur lesquelles l'antigène spécifique est immobilisé par liaison covalente, et
dans lequel l'antigène spécifique est un mélange d'une pluralité de substances et le mélange d'une pluralité de substances est un lysat dérivé d'un micro-organisme.

5. Réactif de dosage d'anticorps selon la revendication 4, dans lequel l'antigène spécifique contient au moins une substance ayant un poids moléculaire de 5000 ou plus.

6. Réactif de dosage d'anticorps selon la revendication 4 ou 5, dans lequel les particules porteuses insolubles sont des particules de latex.

7. Kit de dosage comprenant :
le réactif de dosage d'anticorps selon l'une quelconque des revendications 4 à 6 ; et
un diluant.
